# EUROPEAN PATENT APPLICATION

(11) **EP 4 552 645 A1**
(43) Date of publication of application: **14.05.2025**
(21) Application number: 22949432.3
(22) Date of filing: 30.06.2022
(51) Int. Cl.: A61K 39/00, A61K 39/13, A61K 39/145, A61K 39/165, A61K 39/20, A61K 39/215, A61K 39/235, A61K 39/245, A61K 39/39, A61P 31/12, A61P 37/04, A61K 47/18, A61K 47/42

(54) **VACCINE COMPOSITION FOR SUBLINGUAL ADMINISTRATION**

(71) Applicant: EPS INNOVATIVE MEDICINE (JAPAN) CO., LTD., Tokyo 162-0821 (JP)
(72) Inventor: YAMAMOTO Tetsuro, Tokyo 162-0821 (JP); TANJI Masanori, Tokyo 162-0821 (JP)
(74) Representative: Müller-Boré & Partner Patentanwälte PartG mbB
(86) International application number: PCT/JP2022/026343
(87) International publication number: WO 2024/004159

(57) **Abstract**

This is to provide a vaccine composition suitable for sublingual administration, a method of producing vaccine and a method of administering vaccine.

This is to provide a vaccine composition for sublingual administration in a subject comprising an immune antigen and an adjuvant.

## Description

### TECHNICAL FIELD

The present invention relates to a vaccine composition for sublingual administration.

### BACKGROUND ART

The efficacy of a vaccine may be affected not only by the selection of an immune antigen and an adjuvant but also by the route of administration. For viruses such as influenza virus and coronaviruses, etc., that infect epithelial cells of bronchi and lungs, it is more important to induce secretory IgA antibodies specific for viral antigen in the upper respiratory mucosa than IgG antibodies in blood (Non-Patent Document 1). Recently, in contrast to a blood IgG antibody/systemic immune responses, vaccine administration through the nasal or oral route has been developed which induces a secreted IgA antibody/mucosal immune response (Non-Patent Document 2). On the other hand, there has been little progress in the development of practical protein vaccines for sublingual administration. In primates, including humans and monkeys, the sublingual region has a large space and is easier to administer vaccines than the nasal cavity, but the effectiveness of vaccines administered sublingually has not been confirmed (Non-Patent Documents 3 and 4).

### PRIOR ART DOCUMENTS

### NON-PATENT DOCUMENTS

Non-Patent Document 1: A. Ainai, et al. Human immune responses elicited by an intranasal inactivated H5 influenza vaccine, Microbiology and Immunology. 2020; 64:313-325.
Non-Patent Document 2: R. Mudgal, et al. Prospects for mucosal vaccine: shutting the door on SARS-CoV-2, HUMAN VACCINES IMMUNOTHERAPEUTICS 2020, VOL. 16, NO. 12, 2921-2931.
Non-Patent Document 3: R. S. Veazey, et al, Evaluation of mucosal adjuvants and immunization routes for the induction of systemic and mucosal humoral immune responses in macaques, Human Vaccines & Immunotherapeutics 11:12, 2913--2922; December 2015.
Non-Patent Document 4: Alan D. Curtis II, et al, A simultaneous oral and intramuscular prime/sublingual boost with a DNA/Modified Vaccinia Ankara viral vector-based vaccine induces simian immunodeficiency virus-specific systemic and mucosal immune responses in juvenile rhesus macaques, J Med Primatol. 2018 October; 47(5): 288-297.

### SUMMARY OF THE INVENTION

### PROBLEMS TO BE SOLVED BY THE INVENTION

This is to provide a vaccine composition suitable for sublingual administration, a vaccine production method, and a vaccine administration method.

### MEANS TO SOLVE THE PROBLEMS

The inventors of the present application have newly found, as a result of intensive studies, a vaccine composition suitable for upper respiratory tract mucosal administration, particularly for sublingual administration.

The inventions of the present application include the following [1] to [18C].
[1] A vaccine composition for sublingual administration to a subject, which comprises an immune antigen and an adjuvant.
[2] The vaccine composition described in [1], which further comprises a mucolytic agent.
[3] The vaccine composition described in [1], which is administered after sublingually administering a mucolytic agent to the subject.
[4] The vaccine composition described in [2] or [3], wherein the mucolytic agent is selected from the group consisting of a proteolytic enzyme, a protein denaturing agent, a deoxyribonuclease, a reducing agent and a calcium chelating agent.
[5] The vaccine composition described in [4], wherein the reducing agent is cysteine or a derivative thereof.
[6] The vaccine composition described in [5], wherein the derivative of cysteine is N-acetylcysteine (NAC) or carbocysteine.
[7] The vaccine composition described in [4], wherein the proteolytic enzyme is trypsin, papain or bromelain.
[8] The vaccine composition described in [4], wherein the protein denaturing agent is urea or guanidinium hydrochloride.
[9] The vaccine composition described in [4], wherein the deoxyribonuclease is human DNaseI.
[10] The vaccine composition described in [4], wherein the calcium chelating agent is bicarbonate or ethylene glycol-bis(2-aminoethyl ether)-N,N,N',N'-tetraacetic acid (EGTA).
[11] The vaccine composition described in [1], wherein the immune antigen is derived from varicella virus, measles virus, mumps virus, poliovirus, rotavirus, influenza virus, adenovirus, herpes virus, rubella virus, SARS virus or HIV.
[12] The vaccine composition described in [1], wherein the immune antigen comprises a spike protein receptor binding domain of SARS-CoV-2.
[13] The vaccine composition described in [1], wherein the adjuvant comprises a Toll-like receptor (TLR) ligand, squalene or an aluminum salt.
[14] The vaccine composition described in [13], wherein the TLR ligand is double-stranded RNA (dsRNA).
[15] The vaccine composition described in [14], wherein the dsRNA is Poly(I:C).
[16] The vaccine composition described in [1], wherein a weight ratio of the comprised immune antigen to the adjuvant is 10:400 to 400:400, preferably 20:400 to 200:400, and more preferably 30:400 to 150:400.
[17] The vaccine composition described in [1], wherein the immune antigen is comprised in an amount of 10 to 400 µg, preferably 20 to 200 µg, and more preferably 30 to 150 µg.
[18] The vaccine composition described in [1], which is administered to the subject three times at 4-week intervals.

[1A] A method for imparting immunogenicity to an immune antigen in a subject, the method comprising
   1) sublingually administering an immune antigen and an adjuvant to the subject.
[2A] The method described in [1A], wherein the immune antigen and the adjuvant are sublingually administering together with a mucolytic agent.
[3A] The method described in [1A], wherein the immune antigen and the adjuvant are administered after sublingually administering a mucolytic agent to the subject.
[4A] The method described in [2A] or [3A], wherein the mucolytic agent is selected from the group consisting of a proteolytic enzyme, a protein denaturing agent, a deoxyribonuclease, a reducing agent and a calcium chelating agent.
[5A] The method described in [4A], wherein the reducing agent is cysteine or a derivative thereof.
[6A] The method described in [5A], wherein the derivative of cysteine is N-acetylcysteine (NAC) or carbocysteine.
[7A] The method described in [4A], wherein the proteolytic enzyme is trypsin, papain or bromelain.
[8A] The method described in [4A], wherein the protein denaturing agent is urea or guanidinium hydrochloride.
[9A] The method described in [4A], wherein the deoxyribonuclease is human DNaseI.
[10A] The method described in [4A], wherein the calcium chelating agent is bicarbonate or ethylene glycol-bis(2-aminoethyl ether)-N,N,N',N'-tetraacetic acid (EGTA).
[11A] The method described in [1A], wherein the immune antigen is derived from a virus selected from varicella virus, measles virus, mumps virus, poliovirus, rotavirus, influenza virus, adenovirus, herpes virus, rubella virus, SARS virus and HIV, wherein preferably, infection with said virus is prevented or treated in the subject by the administration.
[12A] The method described in [1A], wherein the immune antigen comprises a spike protein receptor binding domain of SARS-CoV-2.
[13A] The method described in [1A], wherein the adjuvant comprises a Toll-like receptor (TLR) ligand, squalene or an aluminum salt.
[14A] The method described in [13A], wherein the TLR ligand is double-stranded RNA (dsRNA).
[15A] The method described in [14A], wherein the dsRNA is Poly(I:C).
[16A] The method described in [1A], wherein a weight ratio of the comprised immune antigen to the adjuvant is 10:400 to 400:400, preferably 20:400 to 200:400, and more preferably 30:400 to 150:400.
[17A] The method described in [1A], wherein the immune antigen is comprised in an amount of 10 to 400 µg, preferably 20 to 200 µg, and more preferably 30 to 150 µg.
[18A] The method described in [1A], wherein the immune antigen and the adjuvant are administered to the subject three times at 4-week intervals.

[1B] A combination of an immune antigen and an adjuvant for use in imparting immunogenicity to an immune antigen by sublingual administration in a subject.
[2B] The combination described in [1B], which are sublingually administered together with a mucolytic agent.
[3B] The combination described in [1B], which is administered sublingually after sublingually administering a mucolytic agent to the subject.
[4B] The combination described in [2B] or [3B], wherein the mucolytic agent is selected from the group consisting of a proteolytic enzyme, a protein denaturing agent, a deoxyribonuclease, a reducing agent and a calcium chelating agent.
[5B] The combination described in [4B], wherein the reducing agent is cysteine or a derivative thereof.
[6B] The combination described in [5B], wherein the derivative of cysteine is N-acetylcysteine (NAC) or carbocysteine.
[7B] The combination described in [4B], wherein the proteolytic enzyme is trypsin, papain or bromelain.
[8B] The combination described in [4B], wherein the protein denaturing agent is urea or guanidinium hydrochloride.
[9B] The combination described in [4B], wherein the deoxyribonuclease is human DNaseI.
[10B] The combination described in [4B], wherein the calcium chelating agent is bicarbonate or ethylene glycol-bis(2-aminoethyl ether)-N,N,N',N'-tetraacetic acid (EGTA).
[11B] The combination described in [1B], wherein the immune antigen is derived from a virus selected from varicella virus, measles virus, mumps virus, poliovirus, rotavirus, influenza virus, adenovirus, herpes virus, rubella virus, SARS virus and HIV; wherein preferably, the combination for use in the prevention or treatment of infection with said viruses.
[12B] The combination described in [1B], wherein the immune antigen comprises a spike protein receptor binding domain of SARS-CoV-2.
[13B] The combination described in [1B], wherein the adjuvant comprises a Toll-like receptor (TLR) ligand, squalene or an aluminum salt.
[14B] The combination described in [13B], wherein the TLR ligand is double-stranded RNA (dsRNA).
[15B] The combination described in [14B], wherein the dsRNA is Poly(I:C).
[16B] The combination described in [1B], wherein a weight ratio of the immune antigen and the adjuvant is 10:400 to 400:400, preferably 20:400 to 200:400, and more preferably 30:400 to 150:400.
[17B] The combination described in [1B], wherein the immune antigen is comprised in an amount of 10 to 400 µg, preferably 20 to 200 µg, and more preferably 30 to 150 µg.
[18B] The combination described in [1B], which is administered to the subject three times at 4-week intervals.

[1C] Use of an immune antigen and an adjuvant in the manufacture of a medicament for sublingual administration to impart immunogenicity to an immunizing antigen in a subject.
[2C] The use described in [1C], wherein the medicament comprises a mucolytic agent.
[3C] The use described in [1C], wherein the medicament is administered after administering a mucolytic agent sublingually to the subject.
[4C] The use described in [2C] or [3C], wherein the mucolytic agent is selected from the group consisting of a proteolytic enzyme, a protein denaturing agent, a deoxyribonuclease, a reducing agent and a calcium chelating agent.
[5C] The use described in [4C], wherein the reducing agent is cysteine or a derivative thereof.
[6C] The use described in [5C], wherein the derivative of cysteine is N-acetylcysteine (NAC) or carbocysteine.
[7C] The use described in [4C], wherein the proteolytic enzyme is trypsin, papain or bromelain.
[8C] The use described in [4C], wherein the protein denaturing agent is urea or guanidinium hydrochloride.
[9C] The use described in [4C], wherein the deoxyribonuclease is human DNaseI.
[10C] The use described in [4C], wherein the calcium chelating agent is bicarbonate or ethylene glycol-bis(2-aminoethyl ether)-N,N,N',N'-tetraacetic acid (EGTA).
[11C] The use described in [1C], wherein the immune antigen is derived from a virus selected from varicella virus, measles virus, mumps virus, poliovirus, rotavirus, influenza virus, adenovirus, herpes virus, rubella virus, SARS virus and HIV; wherein preferably, the medicament is a medicament for prevention or treatment of infection with the viruses.
[12C] The use described in [1C], wherein the immune antigen comprises a spike protein receptor binding domain of SARS-CoV-2.
[13C] The use described in [1C], wherein the adjuvant comprises a Toll-like receptor (TLR) ligand, squalene or an aluminum salt.
[14C] The use described in [13C], wherein the TLR ligand is double-stranded RNA (dsRNA).
[15C] The use described in [14C], wherein the dsRNA is Poly(I:C).
[16C] The use described in [1C], wherein a weight ratio of the immune antigen to the adjuvant comprised in the medicament is 10:400 to 400:400, preferably 20:400 to 200:400, and more preferably 30:400 to 150:400.
[17C] The use described in [1C], wherein the immune antigen is comprised in the medicament in amount of 10 to 400 µg, preferably 20 to 200 µg, and more preferably 30 to 150 µg.
[18C] The use described in [1C], wherein the medicament is administered to the subject three times at 4-week intervals.

### EFFECTS OF THE INVENTION

The vaccine composition or medicament for sublingual administration and the methods of administration thereof according to the present invention are effective not only for systemic immune response but also for prevention or treatment of viral infection from the upper respiratory tract mucosa by activating mucosal immunity.

### BRIEF DESCRIPTION OF THE DRAWING

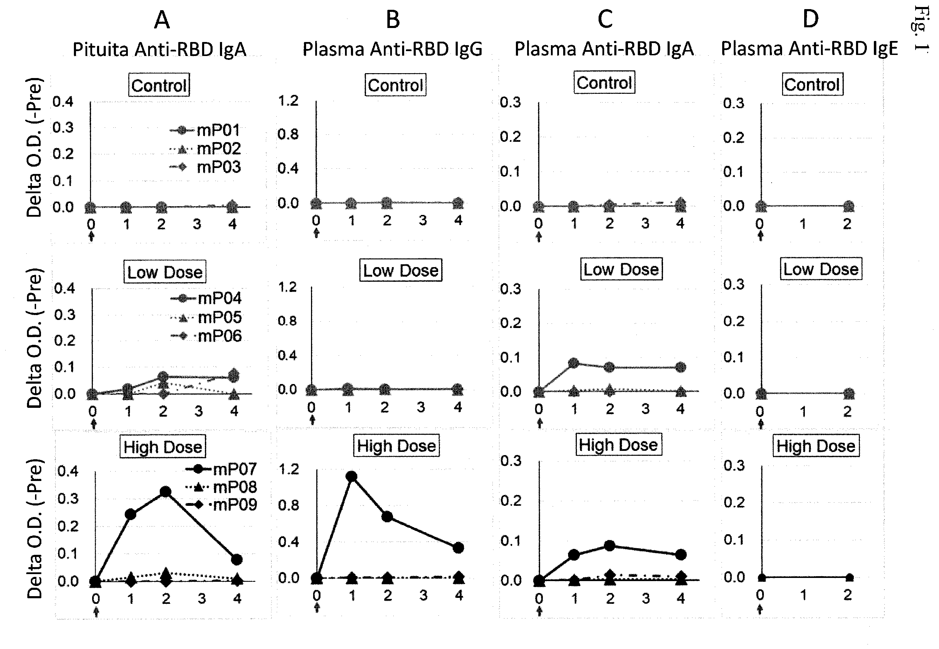

Fig. 1 shows the results of preclinical studies in the primate model cynomolgus monkey with a sublingually administered vaccine comprising SARSCoV2RBD antigen and poly(I:C) adjuvant. Fig. 1A: Amount of anti-RBDIgA in saliva; Fig. 1B: Amount of anti-RBDIgG in plasma; Fig. 1C: Amount of anti-RBDIgA in plasma; Fig. 1D: Amount of anti-RBDIgE in plasma.

### EMBODIMENT TO CARRY OUT THE INVENTION

In the present specification, the term "comprising" may comprehend "essentially comprising," "comprising an effective amount of" and "consisting of".

The upper respiratory tract means nasal cavity, paranasal sinuses, pharynx and larynx, although a respiratory tract runs from the nasal cavity to lungs. An upper respiratory tract infection means that reveal symptoms such as nasal discharge, nasal obstruction, and sore throat are caused by viruses and bacteria that enter from the outside (mostly in the air) and adhere to the upper respiratory tract, and multiply (i.e., infect). The upper respiratory tract infection includes acute upper respiratory tract infection (so-called cold syndrome), acute pharyngitis/tonsillitis, acute laryngitis, and acute epiglottitis. On the other hand, lower respiratory tract infection means that viruses infected in the upper respiratory tract migrate from the trachea to the respiratory bronchi and multiply at these sites to reveal symptoms of bronchitis. In the present specification, the upper respiratory tract infection and the lower respiratory tract infections are included in the infection.

One embodiment of the present invention is a vaccine composition or a medicament comprising an immune antigen and an adjuvant. The vaccine composition or the medicament is preferably administered sublingually. The subject to be administered is not particularly limited as long as it is a mammal having an immune response, and is preferably primates and more preferably humans.

In one embodiment of the present invention, the immune antigen (also referred to simply as antigen) is not particularly limited as long as it has antigenicity, and includes not only peptidic antigens (that is, antigen peptides) but also, non-peptidic antigens such as phospholipids and complex carbohydrates (for example, bacterial membrane constitutional components such as mycolic acid, lipoarabinomannan, etc.).

The immune antigen preferably comprises constitutional component derived from pathogens such as viruses.

Such pathogens are not particularly limited to, but may include protozoa (for example, *Plasmodium falciparum, Leishmania* species and *Trypanosoma* species), bacteria (for example, gram-positive cocci, gram-positive rods, gram-negative bacteria and anaerobes), fungi (for example, *Aspergillus, Blastomyces, Candida, Coccidioides, Cryptococcus, Histoplasma, Paracoccidioides* and *Sporothrix*), viruses (for example, varicella virus, measles virus, mumps virus, poliovirus, rotavirus, adenovirus, herpes simplex virus, papillomavirus, respiratory syncytiaviruses, poxviruses, HIV, influenza viruses and coronaviruses such as SARS-CoV and SARS-CoV2), intracellular parasites (for example, *Chlamydiaceae, Mycoplasmadaceae, Acholeplasmaceae* and *Rickettsiaceae*), and helminths (for example, nematodes, trematodes and tapeworms), etc. Since, among these, not only liquid immunity but also mucosal immunity is expected against viruses infecting the upper respiratory tract (for example, varicella virus, measles virus, mumps virus, poliovirus, rotavirus, influenza viruses, adenovirus, herpes viruses, rubella virus, SARS virus, or HIV), they are suitable for the present invention. Therefore, the present invention can be applied to prevention and treatment of infection by these infectious pathogens such as protozoa, bacteria, fungi, viruses, intracellular parasites, and helminths, etc. (i.e., to suppression of growth of these infectious pathogens in the body).

The "antigen peptide" used in the present specification is not particularly limited as long as it is a peptide composed of two or more amino acids (including those composed of more than 50 amino acids) that can serve as an antigen, and may be of natural origin or synthetic origin, or commercially available. The antigen peptide may contain a full-length amino acid sequence of gene product or a partial amino acid sequence thereof. For example, it may include antigen peptides (containing their full-length sequences and partial sequences) derived from the above-mentioned infectious pathogens such as protozoa, bacteria, fungi, viruses, intracellular parasites, and helminths. For example, the SARSCoV-2 spike protein receptor binding domain (RBD) corresponds to the antigen peptide.

The antigen peptide may be subjected to any processing or modification (for example, phosphorylation or glycosylation).

In one embodiment of the present invention, the adjuvant is a substance used to enhance the effect (immunogenicity) of an immune antigen when administered together with it. The adjuvant is not particularly limited to, but may include those comprising an aluminum salt (for example, AS04), those comprising squalene (for example, MF59, AS03, AddaS03) or those comprising Toll-like receptor (TLR) ligand, etc.

The TLR ligand is not particularly limited to, but may include triacyl lipoproteins (as TLR1/2 ligand); peptidoglycan, lipoarabinomannan and porin (as TLR2 ligand); double-stranded RNA (as TLR3 ligand) such as Poly(I:C); lipopolysaccharide (as TLR4 ligand); flagellin (as TLR5 ligand); diacyl lipoprotein, zymosan (as TLR2/6 ligand) and single-stranded RNA (as TLR7/8 ligand); and CpG-DNA (as TLR9 ligand), etc. By binding these ligands to the TLRs of immune cells, the TLRs are homodimerized or heterodimerized to transmit signals into and activate the immune cells (i.e., to elicit immune response), and, as a result, production of inflammatory cytokine and production of type I interferon are induced.

In one embodiment of the present invention, the vaccine composition or the medicament may comprise the immune antigen in an amount of 10 to 400 µg, preferably 20 to 200 µg, and more preferably 30 to 150 µg per one administration dose.

In one embodiment of the present invention, a weight ratio of the immune antigen to the adjuvant may be 10:400 to 400:400, preferably 20:400 to 200:400, and more preferably 30:400 to 150:400.

In one embodiment of the present invention, the vaccine composition or the medicament may comprise a mucolytic agent, which may be administered after administration of the mucolytic agent or simultaneously with administration of the same.

Mucus covers the epithelial surfaces of the respiratory tract including the nasal cavity and oral cavity, digestive organs such as the stomach and intestines, etc., vagina, joints, and eyes, etc., forming a barrier against foreign substances and pathogens. Mucin, the main structural component present in mucus, is a macromolecule formed by sugar chain modification of apomucin, which is composed of a protein backbone having an intermittent cysteine-rich region that participates in mucin-mucin interactions via disulfide bonds and a central region containing tandem repeats rich in threonine, serine, and proline. (hereinafter, the above-mentioned barrier may be sometimes referred to as a mucin layer, a mucus gel, or a mucus layer).

A mucolytic agent is a drug that reduces the viscosity of mucus gel. The mucolytic agent is not particularly limited to, but may include proteolytic enzymes, protein-denaturing agents, deoxyribonuclease, reducing agents, and calcium chelating agents are included. The mucolytic agent can increase permeability of the mucus gel present under the tongue to make it easier for immune antigens and adjuvants to be absorbed into the body from the sublingual region, thereby a mucosal immune response and/or a systemic immune response can be induced.

Since mucus is a complex network of cross-linked proteins, by utilizing proteolytic enzymes, peptide bonds are broken and non-glycosylated mucin domains are cleaved to degrade the mucin protein backbone and/or proteins in the mucus gel, thereby permeability of the mucus gel can be increased.

Such proteolytic enzymes may include trypsin, papain or bromelain.

In the present invention, in the proteolytic enzymes, glycosidases that degrade glycosylation of mucins may be included. By degrading glycosylation, the same effect as proteolytic enzymes can be obtained.

Since mucus also comprises DNA, which is a macromolecule, permeability of the mucus gel can be increased by degrading DNA in the mucus.

Such deoxyribonuclease (DNA degrading enzyme) may include human DNaseI (enzyme number 3.1.21.1) or its active fragments.

The permeability of mucus gels can be increased by reducing agents that reduce and cleave disulfide bonds between mucins in mucus.

Such reducing agents may include cysteine or its modifications. Modifications of cysteine may include N-acetylcysteine (NAC), S-carboxymethylcysteine (carbocysteine), etc.

The permeability of mucus gels can be increased by protein-denaturing agents that break the inter- and intramolecular hydrogen bonds between mucin molecules in the mucus.

Such protein-denaturing agents may include urea and guanidinium hydrochloride.

The permeability of the mucin layer in mucus can be increased by hydrating, swelling, and dispersing the mucin layer with calcium chelating agents.

Such calcium chelating agents may include bicarbonate and ethylene glycolbis(2-aminoethyl ether)-N,N,N',N'-tetraacetic acid (EGTA).

In one embodiment of the invention, it is preferred that the vaccine composition is administered to a subject at least three times. The administration interval is preferably 3 to 5 weeks interval, and preferably 4 weeks interval.

Further, in order to elicit an immune response, it may be administered at least once more.

In one embodiment of the invention, the vaccine composition or the medicament may further comprise a pharmaceutically acceptable, carrier; antioxidant; preservative; colorant; diluent; emulsifier; suspending agent; solvent; filler; bulking agent; buffering agent; delivery vehicle; diluent; excipient, etc.

Hereinafter, the present invention is described in detail by referring to Examples, but the present invention is not limited by these Examples.

### EXAMPLES

### Example 1

A preclinical test was conducted and verified in a primate model cynomolgus monkey for a sublingual vaccine comprising SARSCoV2RBD antigen and poly(I:C) adjuvant.

### 1. Materials and methods

### 1.1. Reagents and antibodies

With regard to N-acetylcysteine (NAC), bovine serum albumin (BSA), sodium caseinate, sodium azide (NaN₃) and polyoxyethylene sorbitan monolaurate 20 (Tween 20), the products were purchased from FUJIFILM Wako Pure Chemical Corporation and used. Phosphate buffered saline (PBS) was purchased from Nissui (Japan); polyester disinfection cotton was from JCB Industry Limited (Japan); filter spin column was from Notgen Biotech; Nunc-immune module, F8 Maxisorp was from Thermo Fisher Scientific; Streptavidin-HRP conjugate (SA-HRP) was from Invitrogen; and Tetramethylbenzidine (TMB) was from Sigma-Aldrich, respectively, and used.

Poly(I:C) (HMW vaccine grade) was purchased from InvivoGen; recombinant SARSCoV-2 spike protein receptor binding domain (RBD) was from Creative Diagnostics; and ELAST ELISA sensitization system was from PerkinElmer Inc., respectively, and used.

Biotin-labeled (BT)-monkey IgA antibody was purchased from Mabtech; BT-monkey IgA (α chain) antibody was from Merck; HRP-human IgG antibody was from EY Laboratories; and BT IgE antibody was from Bio-Rad Laboratories Inc., respectively, and used.

### 1.2. Animals

Nine cynomolgus monkeys (males and females; 12.1 to 20.6 years old) were used. The nine cynomolgus monkeys were divided into three groups, each group consisting of three monkeys: a control group (mP01 to 03), a low-dose group mP04 to 06) and a high-dose group (mP07 to 09).

### 1.3. Vaccine administration and sampling

Before vaccine administration, a mixture of medetomidine and ketamine was injected as an anesthetic to put the monkey to sleep, and then the monkey was treated with cotton soaked in 1% NAC for 5 minutes to further break down the mucin layer on the sublingual surface, followed by washed extensively with PBS to remove NAC. The sublingual surface was then wiped gently with dry cotton to remove moisture.

To the sublingual region of the monkeys in the control group (Control), 0.7 ml of the test solution comprising only 400 µg poly(I:C) adjuvant was administered with a pipette per each monkey and allowed to stand for 1 minute.

To the sublingual region of the monkeys in the low-dose group (Low Dose), 0.7 ml of the test solution comprising 30 µg RBD antigen and 400 µg poly(I:C) adjuvant was administered with a pipette and allowed to stand for 1 minute.

To the sublingual region of the monkeys in the high-dose group (High Dose), 0.7 ml of the test solution comprising 150 µg RBD antigen and 400 µg poly(I:C) adjuvant was administered with a pipette and allowed to stand for 1 minute.

Thereafter, the monkeys in each group were injected with atipamezole to induce arousal.

Administration of the above-mentioned vaccine composition was carried out three times at four-week intervals, and administration of a booster for sample collection for ELISA measurement was carried out 15 weeks after the third vaccination. Blood collection and nasal fluid collection were carried out under the above-mentioned anesthesia. The blood was centrifuged and the obtained plasma sample was applied to RBD-specific IgA, IgG, IgG and IgE antibody measurements. In addition, the nasal secretion sample was collected by absorbing into a polystyrene fiber swab, collected by centrifugation using a spin column, and used for ELISA measurement of RBD-specific secreted IgA.

### 1.4. ELISA

A Nunc immunomodule plate was coated with 5 µg RBD antigen (100 µl), incubated at 37°C for 1 hour, and then allowed to stand at 4°C overnight. After this plate was washed with 0.05% Tween 20-containing PBS, a blocking agent of PBS containing 1% sodium caseinate and 0.02% NaN₃ was added, and after incubating at 37°C for 1 hour, it was allowed to stand at 4°C overnight.

Nasal secretion and plasma samples were diluted 100 to 500-fold and used as ELISA sample. After removing the blocking agent, the same amount of 1 M aqueous sodium chloride solution was added to 50 µl of the ELISA sample (final concentration 0.5 M) to prevent nonspecific reaction. After incubating at 37°C for 1 hour, the above-mentioned sample was removed and the plate was washed with PBS containing 0.05% Tween 20. Next, an appropriately diluted antibody for detection (BT monkey IgA antibody, BT monkey IgA (alpha chain) antibody, HRP human IgG antibody or BT IgE antibody) was added and incubated at 37°C for 1 hour. After washing the plate, it was subjected to sensitization treatment with ELAST System containing SA-HRP and. According to this sensitization, the sensitivity of ELISA was increased by 10- to 30-fold.

After washing the sensitized plate with PBS containing 0.05% Tween 20, diluted SA-HRP was added thereto and it was incubated at 37°C for 1 hour. Color development was carried out using TMB (3,3',5,5'-tetramethylbenzidine) and the reaction was stopped with sulfuric acid. Absorptions at 450 nm and 600 nm were measured with iMark microplate reader (Bio-Rad Laboratory).

### 2. Results

The results are shown in Fig. 1. RBD-specific IgA antibody antibodies were observed in the nasal secretions of both the low-dose (30 µg/head) and high-dose (150 µg/head) vaccine administration groups, it has been shown that the production of RBD antigen-specific secretory IgA by mucosal immunity in the nasal cavity and oral cavity is induced by the sublingual vaccine according to the present invention. Further, IgG and IgA were detected in the plasma, but IgE was not detected. This indicates that liquid immunity against RBD was acquired by the vaccine composition for sublingual administration according to the present invention, but there was no side reaction accompanied by an allergic response.

Further, even when the vaccine composition for sublingual administration according to the present invention was administered, no redness around the administration site, edema, weight loss, or anorexia, etc., was observed.

### Example 2

A preclinical test in a primate model cynomolgus monkey is carried out and verified for a sublingual vaccine comprising SARSCoV2RBD antigen and AddaS03 the adjuvant or poly(I:C) adjuvant.

### 1. Materials and methods

### 1.1. Reagents and antibodies

The same materials used in Example 1 are used.

AddaS03 is purchased from InvivoGen.

### 1.2. Animals

Thirteen cynomolgus monkey are used. The animals are divided into 3 groups: control group (B1: mP01 to 03), AddaS03 adjuvant administration group (mB2: P04 to 08) and poly(I:C) adjuvant administration group (B3: mP09 to 13) (Table 1).

**[Table 1]**

| Experiment group | Administered substance | Adjuvant administered dose (µg or µL/head) | Antigen administered dose (µg/head) | Administered liquid amount (µL/head) | Administration route | Number of example | Animal number |
|---|---|---|---|---|---|---|---|
| B1 | Control Medium (PBS) alone | 0 | 0 | 500 | sublingually | 3 | nP01, 02, 03 |
| B2 | AddaS03 Adjuvant + antigen (RBD) | 250 µL | 150 | 500 | sublingually | 5 | **nP04,** 05, 06, 07, 08 |
| B3 | Poly(I:C) adjuvant + antigen (RBD) | 400 µg | 150 | 500 | sublingually | 5 | nP09, 10, 11, 12, 13 |

### 1.3. Vaccine administration and sampling

Before vaccine administration, a mixture of medetomidine and ketamine was injected as an anesthetic to put the monkey to sleep, and then the monkey was treated with cotton soaked in 1.5% NAC for 5 minutes to further break down the mucin layer on the sublingual surface of the monkey, followed by washed extensively with physiological saline to remove NAC. The sublingual surface is then wiped gently with dry cotton to remove moisture.

To the sublingual region of the monkeys in the group B1, only 500 µl of PBS is administered with a pipette per each monkey and allowed to stand for 5 minutes or longer.

To the sublingual region of the monkeys in the group B2, 500 µl of the test solution comprising 150 µg RBD antigen and 200 µl AddaS03 adjuvant is administered with a pipette and allowed to stand for 5 minutes or longer.

To the sublingual region of the monkeys in the group B3, 500 µl of the test solution comprising 150 µg RBD antigen and 400 µg poly(I:C) adjuvant is administered with a pipette and allowed to stand for 5 minutes or longer.

Thereafter, monkeys in each group are injected with atipamezole to induce arousal.

Administration of the above-mentioned vaccine was carried out three times at four-week intervals, and administration of a booster for sample collection for ELISA measurement is carried out 15 weeks after the third vaccination.

Blood samples and saliva and nasal washes are collected under anesthesia before the vaccine administration and every 2 weeks after the administration. If the vaccine administration date and the collection date are on the same day, the collection is carried out before the vaccine administration. Saliva is collected by leaving a swab in the oral cavity for 5 minutes or longer. For the nasal cavity wash, 200 µL of PBS is sprayed into the left and right nasal cavities using a sprayer (100 µL/l push), held horizontally, and the wash solution is collected from the nostrils after a few minutes.

### 1.4. ELISA

In the same manner as in Example 1, RBD-specific IgA, IgG and IgE antibodies in each sample are detected and measured.

### 1.5. Cytokines in blood

The anti-viral effect of the vaccine and inflammatory response is evaluated by detecting and measuring IL-12, type I interferon (IFN), IFN-γ (type II IFN) and IL-17 in the collected blood samples (serum and plasma).

### 1.6. Measurement of NK activation and examination of gene expression of safety evaluation index

GranzymeB (GzmB) gene expression level, which is an indicator of NK cell (natural killer cell) activation, in peripheral blood mononuclear cells (PBMC) from the collected blood sample is detected and measured. As other safety evaluation index genes, the expression levels of pro-inflammatory response and IFN response-related genes are detected and measured.

### 1.7. DNA microarray analysis using white blood cells (WBC)

RNA is isolated from leukocytes in the collected blood sample and used for DNA microarray analysis.

### 1.8. Determination of CRP in blood and hematological and blood biochemical tests

CRP in the collected blood samples is detected and measured. Red blood cell count (RBC), hemoglobin (HGB), hematocrit value (HCT), mean corpuscular volume (MCV), mean corpuscular hemoglobin content (MCH), mean corpuscular hemoglobin concentration (MCHC), platelet count (PLT), reticulocyte rate (RET), white blood cell count (WBC), and percentage by white blood cell type (DIFFERENTIAL WHITE BLOOD CELL PERCENTAGES) [lymphocyte (LYMPH), neutrophil (NEUT), monocyte (MONO), eosinophil (EO), basophil (BASO)] ratio in the blood samples collected as hematological tests are detected and calculated.

As blood biochemical tests, AST amount, ALT amount, ALP amount, γ-GTP amount, total bilirubin amount, urea nitrogen amount, creatinine amount, glucose amount, total cholesterol amount, phospholipid amount, neutral fat amount, total protein amount, albumin amount, albumin (A)/ globulin (G) ratio, LDH amount, inorganic phosphorus amount, calcium amount, magnesium amount, sodium amount, potassium amount, and chlorine amount in the collected blood samples are detected and calculated.

### INDUSTRIAL APPLICABILITY

The vaccine composition or the medicament for sublingual administration according to the present invention is effective not only for systemic immune response but also for prevention or treatment of viral infection from the upper respiratory tract mucosa by activating mucosal immunity.

## Claims

1. A vaccine composition for sublingual administration to a subject, which comprises an immune antigen and an adjuvant.

2. The vaccine composition according to Claim 1, which further comprises a mucolytic agent.

3. The vaccine composition according to Claim 1, which is administered after sublingually administering a mucolytic agent to the subject.

4. The vaccine composition according to Claim 2 or 3, wherein the mucolytic agent is selected from the group consisting of a proteolytic enzyme, a protein denaturing agent, a deoxyribonuclease, a reducing agent and a calcium chelating agent.

5. The vaccine composition according to Claim 4, wherein the reducing agent is cysteine or a derivative thereof.

6. The vaccine composition according to Claim 5, wherein the derivative of cysteine is N-acetylcysteine (NAC) or carbocysteine.

7. The vaccine composition according to Claim 4, wherein the proteolytic enzyme is trypsin, papain or bromelain.

8. The vaccine composition according to Claim 4, wherein the protein denaturing agent is urea or guanidinium hydrochloride.

9. The vaccine composition according to Claim 4, wherein the deoxyribonuclease is human DNaseI.

10. The vaccine composition according to Claim 4, wherein the calcium chelating agent is bicarbonate or ethylene glycol-bis(2-aminoethyl ether)-N,N,N',N'-tetraacetic acid (EGTA).

11. The vaccine composition according to Claim 1, wherein the immune antigen is derived from varicella virus, measles virus, mumps virus, poliovirus, rotavirus, influenza virus, adenovirus, herpes virus, rubella virus, SARS virus or HIV.

12. The vaccine composition according to Claim 1, wherein the immune antigen comprises a spike protein receptor binding domain of SARS-CoV-2.

13. The vaccine composition according to Claim 1, wherein the adjuvant comprises a Toll-like receptor (TLR) ligand, squalene or an aluminum salt.

14. The vaccine composition according to Claim 13, wherein the TLR ligand is double-stranded RNA (dsRNA).

15. The vaccine composition according to Claim 14, wherein the dsRNA is Poly(I:C).

16. The vaccine composition according to Claim 1, wherein a weight ratio of the comprised immune antigen to the comprised adjuvant is 10:400 to 400:400.

17. The vaccine composition according to Claim 1, wherein the immune antigen is comprised in an amount of 10 to 400 µg.

18. The vaccine composition according to Claim 1, which is administered to the subject three times at 4-week intervals.
